(19) Europäisches Patentamt
European Patent Office
Office européen des brevets

(11) Veröffentlichungsnummer: **0 055 386**
**B1**

(12) # EUROPÄISCHE PATENTSCHRIFT

(45) Veröffentlichungstag der Patentschrift: **26.09.84**

(51) Int. Cl.³: **A 61 K 7/13**, C 07 C 91/12, C 07 C 91/08, C 07 C 91/10

(21) Anmeldenummer: **81109348.3**

(22) Anmeldetag: **30.10.81**

(54) **Haarfärbemittel.**

(30) Priorität: **13.12.80 DE 3047105**

(43) Veröffentlichungstag der Anmeldung: **07.07.82 Patentblatt 82/27**

(45) Bekanntmachung des Hinweises auf die Patenterteilung: **26.09.84 Patentblatt 84/39**

(84) Benannte Vertragsstaaten: **AT BE CH DE FR GB IT LI LU NL SE**

(56) Entgegenhaltungen:
**DE-A- 1 945 451**
**US-A- 3 726 635**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Rose, David, Dr.**
**Holbeinweg 7**
**D-4010 Hilden (DE)**
Erfinder: **Maak, Norbert, Dr.**
**Liebigstrasse 18**
**D-4040 Neuss (DE)**

**EP 0 055 386 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind Mittel zur oxidativen Färbung von Haaren auf Basis von hydroxyalkylierten Alkyl-amino-p-phenylendiaminen als Entwicklerkomponente.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Amino-pyrazolon-derivate, heterocyclische Hydrazone eingesetzt. Als sogenannte Kupplerkomponenten werden m-Phenylendiaminderivate, Phenole, Naphthole, Resorcinderivate und Pyrazolone genannt.

Gute Oxidationshaarfarbstoffkomponenten müssen in erster Linie folgende Voraussetzungen erfüllen :

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen und sie sollen darüber hinaus in toxikologischer und dermatologischer Hinsicht unbedenklich sein.

Die üblicherweise als Entwicklersubstanzen verwendete Verbindungsklasse der substituierten bzw. unsubstituierten p-Phenylendiamine besitzt den Nachteil, daß sie bei einer Reihe von Personen Sensibilisierungen und in deren Gefolge schwere Allergien hervorruft. Die zur Vermeidung dieser dermatologischen Nachteile in neuerer Zeit vorgeschlagenen Entwicklersubstanzen können in ihren anwendungstechnischen Eigenschaften nicht immer voll befriedigen.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß Haarfärbemittel auf Basis von Oxidationsfarbstoffen mit einem Gehalt an hydroxyalkylierten Alkylamino-p-phenylendiaminen der allgemeinen Formel

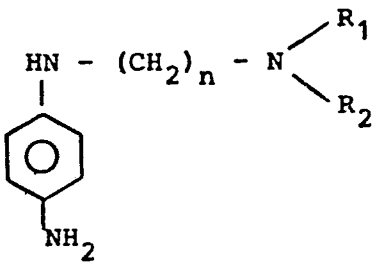

in der n für eine Zahl von 2-4, $R_1$ und $R_2$ für Wasserstoff, einen Mono- oder Polyhydroxyalkylrest mit 2-6 Kohlenstoffatomen stehen, mit der Maßgabe, daß mindestens einer der Reste $R_1$ oder $R_2$ ein Mono- oder Polyhydroxyalkylrest ist, sowie deren anorganischen oder organischen Salzen als Entwicklersubstanzen und den in Oxidationshaarfarben üblichen Kupplerkomponenten den gestellten Anforderungen in besonders hohem Maße gerecht werden.

Bei ihrem Einsatz als Entwicklerkomponenten liefern die erfindungsgemäßen Verbindungen mit den im allgemeinen für die Oxidationshaarfärbung verwendeten Kupplersubstanzen die unterschiedlichsten sehr intensiven Farbtöne und stellen somit eine wesentliche Bereicherung der oxidativen Haarfärbemöglichkeiten dar. Darüber hinaus zeichnen sich die erfindungsgemäßen hydroxyalkylierten Alkylamino-p-phenylendiamine durch sehr gute Echtheitseigenschaften der damit erzielten Färbungen, durch eine gute Löslichkeit im Wasser, eine gute Lagerstabilität und toxikologische, sowie dermatologische Unbedenklichkeit aus.

Die erfindungsgemäß als Entwicklerkomponenten zu verwendenden hydroxyalkylierten Alkylamino-p-phenylendiamine können entweder als solche oder in Form ihrer Salze mit anorganischen oder organischen Säuren, wie z. B. als Chloride, Sulfate, Phosphate, Acetate, Propionate, Lactate, Citrate eingesetzt werden.

Die Herstellung der in den erfindungsgemäßen Haarfärbemitteln als Entwicklerkomponenten einzusetzenden hydroxyalkylierten Alkylamino-p-phenylendiamine kann nach allgemein bekannten Verfahren der organischen Synthese erfolgen. In erster Stufe wird ein N-acetyliertes Alkylendiamin der allgemeinen Formel

$$H_3C\ CO \cdot NH—(CH_2)_n—NH_2$$

in der n die vorgenannte Bedeutung besitzt mit einem Brom-hydroxyalkan umgesetzt. Nach Abspaltung der Acetylgruppe aus dem Umsetzungsprodukt mit Salzsäure wird das erhaltene hydroxyalkylierte Alkylendiamin mit p-Fluornitrobenzol zum N-(p-Nitrophenyl)-N'-hydroxyalkylaminoalkan umgesetzt, welches anschließend zum hydroxyalkylierten Alkylamino-p-phenylendiamin katalytisch hydriert wird. Die Herstellung verläuft dabei zum Beispiel nach folgendem Schema, wobei n und $R_1$ die vorgenannte Bedeutung haben

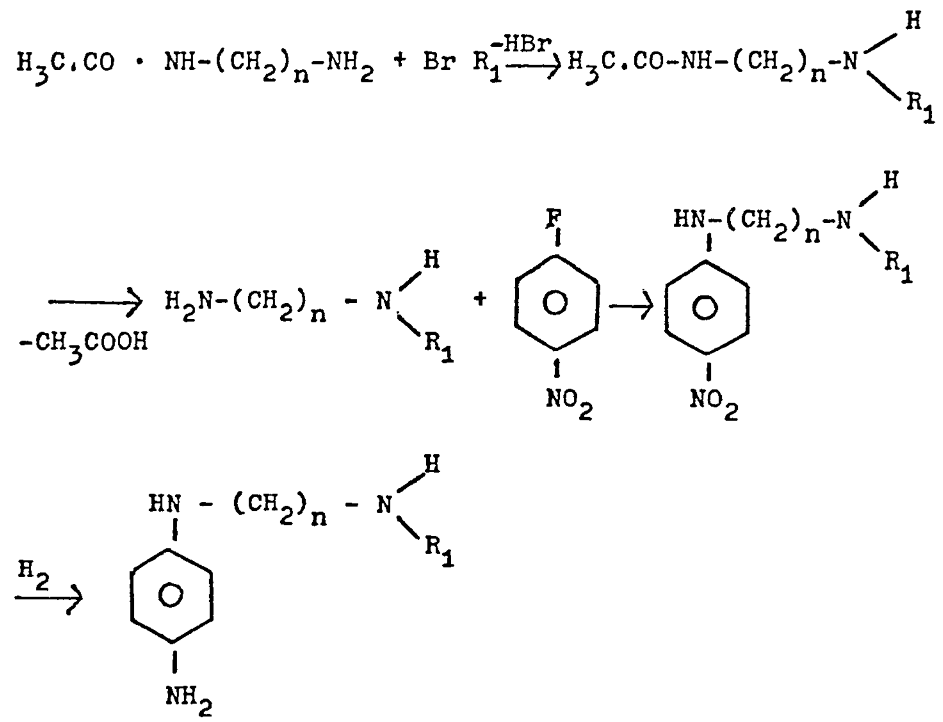


Als Ausgangsmaterial für die erste Stufe können folgende acetylierten Diamine dienen : Ethylendiamin, 1,3-Diaminopropan, 1,4-Diaminobutan. Als Beispiele für Brom-hydroxyalkane, die mit den acetylierten Alkylendiaminen umgesetzt werden können, sind 1-Bromethanol-2, 1-Brompropanol-3, 1-Brombutanol-2, 3-Brombutanol-2, 4-Brompentanol-1, 1-Bromhexanol-2, 3-Brompropandiol-1,2, 4-Brom-2-methyl-butandiol-2,3, 2-Methyl-2-bromethyl-propandiol-1,3 zu nennen.

Als in den erfindungsgemäßen Haarfärbemitteln einzusetzende hydroxyalkylierte Alkylamino-p-phenylendiamine sind zum Beispiel N-(p-Aminophenyl)-N'-(β-hydroxyethyl)-1,2-diaminoethan, N',N'-bis-(β-hydroxyethyl)-1,2-diaminoethan, N'-(β-hydroxyethyl)-1,3-diaminopropan, N',N'-bis-(β-hydroxyethyl)-1,3-diaminopropan, N'-(β-hydroxyethyl)-1,4-diaminobutan, N',N'-bis-(β-hydroxyethyl)-1,4-diaminobutan, N'-(3-hydroxypropyl)-1,2-diaminoethan, N',N'-bis-3-hydroxypropyl)-1,2-diaminoethan, N'-(3-hydroxypropyl)-1,3-diaminopropan, N',N'-bis-(3-hydroxypropyl)-1,3-diaminopropan, N',N'-bis-(3-hydroxypropyl)-1,4-diaminobutan, N',N'-bis-(hydroxybutyl)-1,2-diaminoethan, N'-(2-hydroxybutyl)-1,3-diaminopropan, N'-(1,2-dihydroxypropyl)-1,2-diaminoethan, N',N'-bis-(1,2-dihydroxypropyl)-1,2-diaminoethan, N',N'-bis-(1,2-dihydroxypropyl)-1,3-diaminopropan, N',N'-bis-(2-methyl-2,3-dihydroxybutyl)-1,2-diaminoethan, N',N'-bis-(2-methyl-2,3-dihydroxybutyl)-1,3-diaminopropan anzuführen.

Unter den in den erfindungsgemäßen Haarfärbemitteln als Entwicklerkomponenten einzusetzenden Produkten kommt dem N-(p-Aminophenyl)-N',N'-bis-(β-hydroxyethyl)-1,3-diaminopropan, sowohl von der färberischen Qualität, als auch seinen sonstigen anwendungstechnischen Eigenschaften, die größte Bedeutung zu.

Als Beispiele für in den erfindungsgemäßen Haarfärbemitteln einzusetzende Kupplerkomponenten sind α-Naphthol, o-Kresol, m-Kresol, 2,6-Dimethylphenol, 2,5-Dimethylphenol, 3,4-Dimethylphenol, 3,5-Dimethylphenol, Brenzcatechin, Pyrogallol, 1,5- bzw. 1,7-Dihydroxy-naphthalin, 5-Amino-2-methylphenol, Hydrochinon, 2,4-Diamino-anisol, m-Toluylendiamin, 4-Aminophenol, Resorcin, Resorcinmonomethylether, m-Phenylendiamin, 1-Phenyl-3-methylpyrazolon-5, 1-Phenyl-3-amino-pyrazolon-5, 1-Phenyl-3,5-diketo-pyrazolidin, 1-Methyl-7-dimethyl-amino-4-hydroxychinolon-2, 1-Amino-3-acetyl-acetamino-4-nitrobenzol oder 1-Amino-3-cyanacetyl-amino-4-nitro-benzol anzuführen.

In den erfindungsgemäßen Haarfärbemitteln werden die Entwicklerkomponenten im allgemeinen in etwa molaren Mengen, bezogen auf die verwendeten Kupplersubstanzen, eingesetzt. Wenn sich auch der molare Einsatz als zweckmäßig erweist, so ist es jedoch nicht nachteilig, wenn die Entwicklerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden hydroxyalkylierten Alkylamino-p-phenylendiamine als auch die Kupplersubstanz Gemische der vorstehend genannten Kupplerkomponenten darstellen.

Darüber hinaus können die erfindungsgemäßen Haarfärbemittel andere bekannte und übliche Entwicklerkomponenten, sowie auch gegebenenfalls übliche direktziehende Farbstoffe im Gemisch enthalten, falls dies zur Erzielung gewisser Farbnuancen erforderlich ist.

Die oxidative Kupplung, d. h. die Entwicklung der Färbung, kann grundsätzlich wie bei anderen Oxidationshaarfarbstoffen auch, durch Luftsauerstoff erfolgen. Zweckmäßigerweise werden jedoch

chemische Oxidationsmittel eingesetzt. Als solche kommen insbesondere Wasserstoffperoxid oder dessen Anlagerungsprodukte an Harnstoff, Melamin und Natriumborat, sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen, wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1-3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind z. B. Netz- oder Emulgiermittel vom anionischen oder nichtionogenen Typ, wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel, wie Pantothensäure und Cholesterin zu nennen. Die genannten Zusatzstoffe werden dabei in den für diese Zwecke üblichen Mengen eingesetzt, wie z. B. Netz- und Emulgiermittel in Konzentrationen von 0,5-30 Gewichtsprozent und Verdickungsmittel in Konzentrationen von 0,1-25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8-10 erfolgen. Die Anwendungstemperaturen bewegen sich dabei im Bereich von 15 bis 40 °C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet.

Die mit den erfindungsgemäßen Haarfärbemitteln erzielbaren Farbtöne zeigen unter Einsatz unterschiedlicher Entwickler- und Kupplerkomponenten eine große Variationsmöglichkeit, die von braun bis schwarzblau reicht und als Nuancierkomponente zu besonders intensiven natürlichen Farbtönen führt. Die erzielten Färbungen haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn jedoch hierauf zu beschränken.

## Beispiele

Zunächst wird die Herstellung des in den nachfolgenden Beispielen als Entwicklerkomponente einzusetzenden N-(p-Aminophenyl)-N',N'-bis-(β-hydroxyethyl)-1,3-diaminopropan, welches nicht literaturbekannt ist, beschrieben.

1. Stufe : Herstellung des N-(p-Nitrophenyl)-N',N'-bis-(β-hydroxyethyl)-1,3-diaminopropan.

Ein Gemisch von 14,1 g (0,1 Mol) p-Fluornitrobenzol, 16,2 g (0,1 Mol) N-(3-Aminopropyl)-diethanolamin und 10,4 g (0,075 Mol) Kaliumcarbonat wurde in 20 ml Ethanol plus 5 ml Wasser während 6 Stunden in einem Autoklaven auf 150 °C erhitzt. Nach dem Abkühlen wurde in Eiswasser gegossen und der erhaltene Niederschlag mit Essigester extrahiert. Nach dem Trocknen mit Natriumsulfat wurde zur Trockne eingeengt. Es wurden 16 g an N-(p-Nitrophenyl)-N',N'-bis-(β-hydroxyethyl)-1,3-diaminopropan in Form gelber Kristalle vom Schmelzpunkt 52-54 °C erhalten.

2. Stufe : N-(p-Aminophenyl)-N',N'-bis-(β-hydroxyethyl)-1,3-diaminopropan-trihydrochlorid-trihydrat.

15,8 g des N-(p-Nitrophenyl)-N',N'-bis-(β-hydroxyethyl)-1,3-diaminopropan wurden in 200 ml Ethanol in Gegenwart von 5 % Palladium auf Kohle bei 25 °C und 1 atü hydriert. Nach beendeter Wasserstoffaufnahme wurde vom Katalysator abfiltriert, mit verdünnter Salzsäure angesäuert und eingeengt. Die Verbindung wurde als hellbraunes Öl mit einer Ausbeute von 20 g erhalten.

IR-Spektrum ($cm^{-1}$) : 1 630, 1 585, 1 510, 1 465, 1 320, 1 195, 1 070, 1 020, 900, 825, 755

Die erfindungsgemäßen Haarfärbemittel wurden in Form einer Cremeemulsion eingesetzt. Dabei wurden in eine Emulsion aus

10 Gewichtsteilen Fettalkoholen der Kettenlänge $C_{12}$-$C_{18}$
10 Gewichtsteilen Fettalkoholsulfat (Natriumsalz) Kettenlänge $C_{12}$-$C_{18}$
75 Gewichtsteilen Wasser

jeweils 0,01 Mol N-(p-Aminophenyl)-N',N'-bis-(β-hydroxyethyl)-1,3-diaminopropan-trihydrochlorid-trihydrat und der nachstehend aufgeführten Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1 %iger Wasserstoffperoxidlösung als Oxidationsmittel durchgeführt, wobei zu 100 Gewichtsteilen der Emulsion 10 Gewichtsteile Wasserstoffperoxidlösung

gegeben wurden. Die jeweilige Färbecreme mit zusätzlichem Oxidationsmittel wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Als Kupplersubstanzen dienten folgende Verbindungen :

K 1 : m-Aminophenol
K 2 : 3-Isopropylidenamino-2,4-dichlorphenol
K 3 : 2-Chlor-6-methyl-3-aminophenol
K 4 : Resorcinmonomethylether
K 5 : 2,7-Dihydroxynaphthalin
K 6 : 2,4-Dichlor-3-aminophenol
K 7 : 1,3-Bis-(2,4-diaminophenoxy)-propan
K 8 : 2-Chlorresorcin
K 9 : 2-Methylresorcin
K 10 : 2,4-Diaminophenol
K 11 : 5-Amino-2-methylphenol
K 12 : 1,5-Dihydroxynaphthalin
K 13 : Resorcin
K 14 : α-Naphthol
K 15 : 1-Phenyl-3-acetamido-pyrazolon-5

Tabelle 1

| Beispiel | Kuppler | erhaltener Farbton |
|---|---|---|
| 1 | K 1 | violettgrau |
| 2 | K 2 | schwarzblau |
| 3 | K 3 | dunkelviolett |
| 4 | K 4 | türkisgrau |
| 5 | K 5 | grüngrau |
| 6 | K 6 | schwarzblau |
| 7 | K 7 | tintenblau |
| 8 | K 8 | braungrau |
| 9 | K 9 | braungrau |
| 10 | K 10 | graubraun |
| 11 | K 11 | violett |
| 12 | K 12 | blau |
| 13 | K 13 | braun |
| 14 | K 14 | blau |
| 15 | K 15 | rubin |

**Ansprüche** (für die Vertragsstaaten : BE, CH, DE, FR, GB, IT, LI, LU, NL, SE)

1. Haarfärbemittel auf Basis von Oxidationsfarbstoffen, gekennzeichnet durch einen Gehalt an hydroxyalkylierten Alkylamino-p-phenylendiaminen der allgemeinen Formel

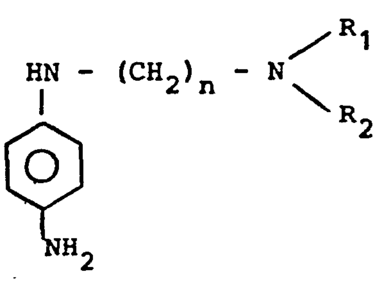

in der n für eine Zahl von 2-4, $R_1$ und $R_2$ für Wasserstoff, einen Mono- oder Polyhydroxyalkylrest mit 2-6 Kohlenstoffatomen stehen, mit der Maßgabe, daß mindestens einer der Reste $R_1$ oder $R_2$ ein Mono- oder Polyhydroxyalkylrest ist, sowie deren anorganischen oder organischen Salzen als Entwicklerkomponenten und den in Oxidationshaarfarben üblichen Kupplersubstanzen.

2. Haarfärbemittel nach Anspruch 1, gekennzeichnet durch einen Gehalt an N-(p-Aminophenyl)-N',N'-bis-(β-hydroxyethyl)-1,3-diaminopropan oder seinen anorganischen oder organischen Salzen als Entwicklerkomponente.

3. Haarfärbemittel nach Anspruch 1 und 2, gekennzeichnet durch einen Gehalt weiterer üblicher Entwicklerkomponenten, sowie gegebenenfalls üblicher direktziehender Farbstoffe.

4. Haarfärbemittel nach Anspruch 1-3, gekennzeichnet durch einen Gehalt an Entwickler-Kuppler-Kombination von 0,2 bis 5 Gewichtsprozent, vorzugsweise 1 bis 3 Gewichtsprozent, bezogen auf das gesamte Mittel.

5. N-(p-Aminophenyl)-N',N'-bis-(β-hydroxyethyl)-1,3-diaminopropan.

**Ansprüche** (für den Vertragsstaat AT)

1. Haarfärbemittel auf Basis von Oxidationsfarbstoffen, gekennzeichnet durch einen Gehalt an hydroxyalkylierten Alkylamino-p-phenylendiaminen der allgemeinen Formel

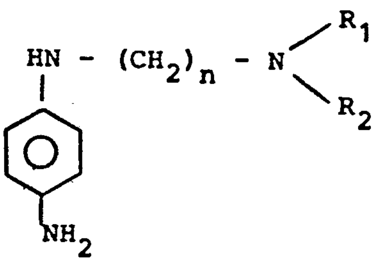

in der n für eine Zahl von 2-4, $R_1$ und $R_2$ für Wasserstoff, einen Mono- oder Polyhydroxyalkylrest mit 2-6 Kohlenstoffatomen stehen, mit der Maßgabe, daß mindestens einer der Reste $R_1$ oder $R_2$ ein Mono- oder Polyhydroxyalkylrest ist, sowie deren anorganischen oder organischen Salzen als Entwicklerkomponenten und den in Oxidationshaarfarben üblichen Kupplersubstanzen.

2. Haarfärbemittel nach Anspruch 1, gekennzeichnet durch einen Gehalt an N-(p-Aminophenyl)-N',N'-bis-(β-hydroxyethyl)-1,3-diaminopropan oder seinen anorganischen oder organischen Salzen als Entwicklerkomponente.

3. Haarfärbemittel nach Anspruch 1 und 2, gekennzeichnet durch einen Gehalt weiterer üblicher Entwicklerkomponenten, sowie gegebenenfalls üblicher direktziehender Farbstoffe.

4. Haarfärbemittel nach Anspruch 1-3, gekennzeichnet durch einen Gehalt an Entwickler-Kuppler-Kombination von 0,2 bis 5 Gewichtsprozent, vorzugsweise 1 bis 3 Gewichtsprozent, bezogen auf das gesamte Mittel.

5. Verfahren zur Herstellung von N-(p-Aminophenyl)-N',N'-bis-(β-hydroxyethyl)-1,3-diaminopropan, gekennzeichnet durch die aufeinanderfolgenden Reaktionsschritte

a) Umsetzung von N-(3-Aminopropyl)-diethanolamin mit p-Fluornitrobenzol zum N-(p-Nitrophenyl)-N',N'-bis-(β-hydroxyethyl)-1,3-diaminopropan ;

b) katalytische Hydrierung des N-(p-Nitrophenyl)-N',N'-bis-(β-hydroxyethyl)-1,3-diaminopropan zum N-(p-Aminophenyl)-N',N'-bis-(β-hydroxyethyl)-1,3-diaminopropan.

**Claims** (for the Contracting States : BE, CH, DE, FR, GB, IT, LI, LU, NL, SE)

1. Hair dyes based on oxidation dyes, characterized by a content of hydroxyalkylated alkylamino-p-phenylene diamines corresponding to the following general formula

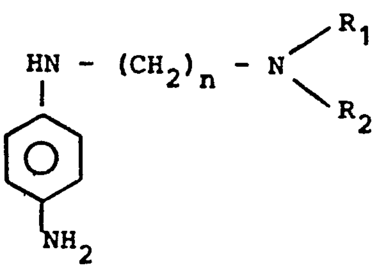

in which n is a number of from 2 to 4, $R_1$ and $R_2$ represent hydrogen or a $C_2$-$C_6$ mono- or polyhydroxyalkyl radical with the proviso that at least one of the radicals $R_1$ or $R_2$ is a mono- or polyhydroxyalkyl radical, and inorganic or organic salts thereof as developer components and the coupler substances normally used in oxidation hair dyes.

2. Hair dyes as claimed in Claim 1, characterized by a content of (N-p-aminophenyl)-N',N'-bis-(β-hydroxyethyl)-1,3-diaminopropane or inorganic or organic salts thereof as developer component.

3. Hair dyes as claimed in Claims 1 and 2, characterized by a content of other standard developer components and, optionally, standard substantive dyes.

4. Hair dyes as claimed in Claims 1 to 3, characterized by a content of developer-coupler combination of from 0.2 to 5 % by weight and preferably from 1 to 3 % by weight, based on the dye as a whole.

5. N-(p-aminophenyl)-N',N'-bis-(β-hydroxyethyl)-1,3-diaminopropane.

**Claims** (for the Contracting State AT)

1. Hair dyes based on oxidation dyes, characterized by a content of hydroxyalkylated alkylamino-p-phenylene diamines corresponding to the following general formula

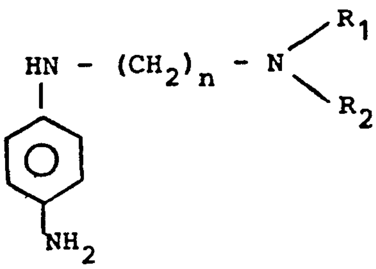

in which n is a number of from 2 to 4, $R_1$ and $R_2$ represent hydrogen or a $C_2$-$C_6$ mono- or polyhydroxyalkyl radical, with the provisoo that at least one of the radicals $R_1$ or $R_2$ is a mono- or polyhydroxyalkyl radical, and inorganic or organic salts thereof as developer components and the coupler substances normally used in oxidation hair dyes.

2. Hair dyes as claimed in Claim 1, characterized by a content of N-(p-aminophenyl)-N',N'-bis-(β-hydroxyethyl)-1,3-diaminopropane or inorganic or organic salts thereof as developer component.

3. Hair dyes as claimed in Claims 1 and 2, characterized by a content of other standard developer components and, optionally, standard substantive dyes.

4. Hair dyes as claimed in Claims 1 to 3, characterized by a content of developer-coupler combination of from 0.2 to 5 % by weight and preferably from 1 to 3 % by weight, based on the dye as a whole.

5. A process for producing N-(p-aminophenyl)-N',N'-bis-(β-hydroxyethyl)-1,3-diaminopropane, characterized by the following successive reaction steps :

a) reacting N-(3-aminopropyl)-diethanolamine with p-fluoronitrobenzene to form N-(p-nitrophenyl)-N',N'-bis-(β-hydroxyethyl)-1,3-diaminopropane ;

b) catalytically hydrogenating the N-(p-nitrophenyl)-N',N'-bis-(β-hydroxyethyl)-1,3-diaminopropane to form N-(p-aminophenyl)-N',N'-bis-(β-hydroxyethyl)-1,3-diaminopropane.

**Revendications** (pour les Etats contractants : BE, CH, DE, FR, GB, IT, LI, LU, NL, SE)

1. Agents de teinture de cheveux, à base de colorants d'oxydation, caractérisés par une teneur en alcoylamino-p-phénylène diamines hydroxyalcoylées de formule

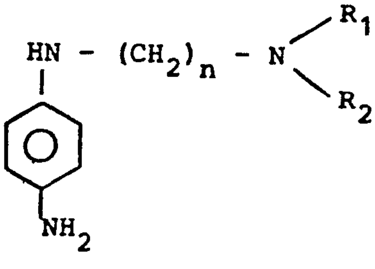

dans laquelle n représente un nombre de 2 à 4, $R_1$ et $R_2$ de l'hydrogène, un radical mono- ou polyhydroxyalcoyle ayant 2 à 6 atomes de carbone, avec comme condition qu'au moins un des radicaux $R_1$ ou $R_2$ soit un radical polyhydroxyalcoyle, ainsi que leurs sels minéraux ou organiques, comme

composants de développement, et en substances de copulation en usage dans les colorants d'oxydation pour cheveux.

2. Agents de teinture de cheveux selon la revendication 1, caractérisés par une teneur en N-(p-aminophényl)-N',N'-bis-(β-hydroxyéthyl)-1,3-diaminopropane ou en ses sels minéraux ou organiques, en tant que composant de développement.

3. Agents de teinture de cheveux selon les revendications 1 et 2, caractérisés par une teneur en d'autres composants de développement usuels, de même qu'éventuellement en des colorants montants directs usuels.

4. Agents de teinture de cheveux selon les revendications 1 à 3, caractérisés par une teneur en une combinaison agent de développement-copulant de 0,2 à 5 % en poids, de préférence de 1 à 3 % en poids, par rapport à l'agent total.

5. N-(p-aminophényl)-N',N'-bis-(β-hydroxyéthyl)-1,3-diaminopropane.

**Revendications** (pour l'Etat contractant AT)

1. Agents de teinture de cheveux, à base de colorants d'oxydation, caractérisés par une teneur en alcoylamino-p-phénylène diamines hydroxyalcoylées de formule

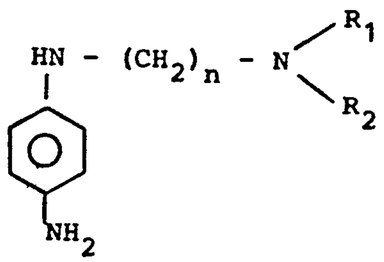

dans laquelle n représente un nombre de 2 à 4, $R_1$ et $R_2$ de l'hydrogène, un radical mono- ou polyhydroxyalcoyle ayant 2 à 6 atomes de carbone, avec comme condition qu'au moins un des radicaux $R_1$ ou $R_2$ soit un radical mono- ou polyhydroxyalcoyle, de même que leurs sels minéraux ou organiques, en tant que composants de développement, et en substances de copulation en usage dans les colorants d'oxydation pour cheveux.

2. Agents de teinture de cheveux selon la revendication 1, caractérisés par une teneur en N-(p-aminophényl)-N',N'-bis-(β-hydroxyéthyl)-1,3-diaminopropane ou en ses sels minéraux ou organiques, en tant que composant de développement.

3. Agents de teinture de cheveux selon les revendications 1 et 2, caractérisés par une teneur en d'autres composants de développement usuels, de même qu'éventuellement en des colorants montants directs usuels.

4. Agents de teinture de cheveux selon les revendications 1 à 3, caractérisés par une teneur en une combinaison agent de développement-copulant de 0,2 à 5 % en poids, de préférence de 1 à 3 % en poids, par rapport à l'agent total.

5. Procédé de préparation du N-(p-aminophényl)-N',N'-bis-(β-hydroxyéthyl)-1,3-diaminopropane, caractérisé par les stades réactionnels successifs de

a) réaction de la N-(3-aminopropyl)-diéthanolamine avec du p-fluoronitrobenzène en N-(p-nitrophényl)-N',N'-bis-(β-hydroxyéthyl)-1,3-diaminopropane ;

b) hydrogénation catalytique du N-(p-nitrophényl)-N',N'-bis-(β-hydroxyéthyl)-1,3-diaminopropane en N-(p-aminophényl)-N',N'-bis-(β-hydroxyéthyl)-1,3-diaminopropane.